# EUROPÄISCHE PATENTSCHRIFT

(11) **EP 2 753 309 B1**
(45) Veröffentlichungstag und Bekanntmachung des Hinweises auf die Patenterteilung: **01.01.2020**
(21) Anmeldenummer: 12758423.3
(22) Anmeldetag: 21.08.2012
(51) Int. Cl.: A61K 9/20, A61K 47/12, A61K 47/10, A61K 31/366

(54) **PHARMAZEUTISCHE ZUSAMMENSETZUNG ZUR ORALEN DARREICHUNG MIT EINEM STATIN**
PHARMACEUTICAL COMPOUND FOR ORAL ADMINISTRATION WITH A STATIN
COMPOSITION PHARMACEUTIQUE POUR L'ADMINISTRATION ORALE DOTÉE D' UNE STATINE

(30) Priorität: 08.09.2011 EP 11180555
(43) Veröffentlichungstag der Anmeldung: 16.07.2014
(73) Patentinhaber: Hexal AG, 83607 Holzkirchen (DE)
(72) Erfinder: TAEUBRICH, Theresa, 83607 Holzkirchen (DE); ROTHER, Patrick, 83607 Holzkirchen (DE)
(74) Vertreter: Baumann, Rüdiger Walter
(86) Internationale Anmeldenummer: PCT/EP2012/066288
(87) Internationale Veröffentlichungsnummer: WO 2013/034436

(56) Entgegenhaltungen:
- EP-A1- 1 992 343
- KR-A- 20050 030 282

## Beschreibung

Die Erfindung betrifft eine pharmazeutische Zusammensetzung zur oralen Darreichung bestehend aus einem Statin, sowie eine(r) erste(n) und eine(r) zweiten antioxidativ wirksame(n) Substanz.

Statine als pharmazeutisch wirksame Substanzen der biopharmazeutischen Klasse II (BCS Klasse II) gelten unter pharmazeutischen Aspekten als instabil und sind besonders anfällig für oxidative Degradation. Um diese zu vermeiden, sind verschiedene Verfahren bzw. Vorgehensweisen bekannt. Hierzu gehören die geeignete Wahl der Darreichungsform, die Zugabe von geeigneten Hilfsstoffen sowie die Auswahl einer geeigneten Verpackung.

Um die oxidative Degradationsrate der Statine zu reduzieren, werden diese zusammen mit antioxidativ wirksamen Substanzen (Antioxidantien) formuliert. Durch die Antioxidantien sowie weitere Hilfsstoffe in der zumeist oralen pharmazeutischen Zusammensetzung wird der Wirkstoff hinreichend stabilisiert.

Nachteilig hierbei ist, dass zur Wirkstoffstabilisierung unter Umständen redundante Maßnahmen ergriffen werden. Beispielsweise weisen die aus dem Stand der Technik bekannten Darreichungsformen eine Hilfsstoffmenge auf, die sich aufgrund ihrer Höhe nachteilig auf Gewicht und Volumen der Darreichungsform sowie die Herstellungskosten auswirkt. Je höher der Hilfsstoffanteil ist, desto geringer ist zudem die Konzentration des Wirkstoffs in der Formulierung. Geringe Wirkstoffkonzentrationen haben wiederum den Effekt, dass der Anteil an Verunreinigungen, wie z.B. Degradationsprodukte des Wirkstoffs, im Verhältnis zum Wirkstoff nachteilig erhöht ist. Daher wird im Allgemeinen eine hohe Wirkstoffkonzentration, d.h. ein geringer Anteil an Hilfsstoffen in der Formulierung angestrebt.

Im Fall der Statine, tritt als wichtiger Aspekt hinzu, dass sich die Löslichkeit bzw. die Auflösungsgeschwindigkeit dieser Substanzen im physiologischen System erhöht, je höher der Anteil an Hilfsstoffen ist, die in der Darreichungsform verwendet werden.

Als Statine im Zusammenhang mit der vorliegenden Erfindung sind pharmazeutisch wirksame Substanzen zu verstehen, die der pharmakologischen Substanzklasse der 3-Hydroxy-3-Methylglutaryl-Coenzym-A-Reduktase-(H MG-CoA-Reduktase -) Inhibitoren angehören. Statine werden hauptsächlich bei Fettstoffwechselstörungen als Cholesterinsenker eingesetzt und weisen bezogen auf alle Medikamente, die den Lipidstoffwechsel beeinflussen, die höchste Potenz auf.

Die WO 2004/010933 A (UNIV LELAND STANFORD JUNIOR [US]; KIM SEUNG [US]; RULIFSON INGRID [US]; HORI YUICHI [US]) 27.07.2003 offenbart eine Zusammensetzung, die neben einem Statin einen Cholesterinabsorptionshemmer, hier Ezetimib, sowie wenigstens eine wirkstoffstabilisierende Hilfssubstanz umfasst. Nachteilig ist, dass die Zusammensetzung nur eine relativ niedrigen Statinanteil aufweist und zudem die Kombination aus Statinen und dem Cholesterinabsorptionshemmer Ezetimib, verglichen mit nur Statine als pharmazeutisch wirksamen Inhaltsstoff aufweisenden Zusammensetzungen, eine höhere Anzahl von gravierenden Nebenwirkungen und damit eine schlechtere Patienten-Compliance zeigt.

KR 20050030282 A (KOREA UNITED PHARM INC) 25.09.2003 offenbart eine Darreichungsform, die gelöstes Simvastatin in einer Weichkapsel aufweist. Nachteilig hierbei erweist sich der hohe Anteil grenzflächenaktiver Substanzen, der zu Steuerung der Bioverfügbarkeit des Statins notwendig ist.

EP 1992343 A1 offenbart eine Simvastatin-Tablette, die zusätzlich Ezetimib enthält.

Die in der im Zusammenhang mit der vorliegenden Erfindung beschriebenen Zusammensetzungen verwendbaren Statine werden anhand ihrer biopharmazeutischen Klasse definiert. Das biopharmazeutische Klassifizierungssystem (BCS) teilt pharmazeutisch wirksame Substanzen hinsichtlich ihrer zu erwartenden Bioverfügbarkeit ein. Die in den erfindungsgemäßen Zusammensetzungen verwendeten Statine sind in die biopharmazeutische Klasse II eingeordnet und weisen eine niedrige Löslichkeit bei hohem Permeationsvermögen auf. Die Resorption wird durch die Löslichkeit und/oder die Lösungsgeschwindigkeit der Arzneistoffe kontrolliert.

Aufgabe der vorliegenden Erfindung war es, die Nachteile bekannter pharmazeutischer Zusammensetzungen zu überwinden und eine pharmazeutische Zusammensetzung bereit zu stellen, in welcher ein Statin in pharmazeutisch annehmbarer Stabilität und Bioverfügbarkeit/Löslichkeit vorliegt und die bei hohem Anteil an pharmazeutisch wirksamer Substanz mit niedrigem Gewicht bzw. Volumen kostengünstig und bevorzugt als feste pharmazeutische Darreichungsform hergestellt werden kann. Insbesondere war es Aufgabe der Erfindung, eine pharmazeutische Zusammensetzung mit einem Statin bereitzustellen, in welcher die Art und Menge der Hilfsstoffe sowie das Wirkstoff-Hilfsstoff-Mengenverhältnis gegenüber bekannten Formulierungen optimiert ist.

Diese Aufgabe wird gelöst durch Bereitstellen einer pharmazeutischen Zusammensetzung bestehend aus
(i) 20 Gewichts-% Simvastatin,
(ii) 0,04 Gewichts-% Butylhydroxyanisol,
(iii) 1,4 Gewichts-% Zitronensäure,
(iv) 20 Gewichts-% Stärke
(v) 30.7 Gewichts-% Lactose Monohydrat,
(vi) 25.0 Gewichts-% Mikrokristalline Cellulose,
(vii) 0.9 Gewichts-% Magnesiumstearat,
(viii) 2 Gewichts-% Filmbeschichtung;
wobei die pharmazeutische Zusammensetzung als Tablette gepresst vorliegt und die Tablette ein Gesamtgewicht von 200 mg aufweist.

Es wurde festgestellt, dass sich bei Verwendung nur einer antioxidativ wirksamen Substanz mit einem niedrigem Anteil an der Formulierung bereits eine signifikante Steigerung der Wirkstoffstabilität erzielen lässt, verglichen mit einer Formulierung, welche keine antioxidativen Substanzen enthält. Überraschend zeigte sich, dass die erreichte Wirkstoffstabilität im Vergleich zu den aus dem Stand der Technik bekannten Formulierungen, bei denen der Wirkstoff herkömmlicherweise durch Verwendung von zwei und mehr antioxidativen Substanzen in zum Teil weit höheren Anteilen stabilisiert wird, teilweise erhöht, zumindest aber gleichwertig ist. Die erzielte Wirkstoffstabilität erfüllt und übertrifft damit pharmazeutische Anforderungen. Die erfindungsgemäße pharmazeutische Zusammensetzung weist durch eine gegenüber bekannten Zusammensetzungen verringerte Menge an verwendeten Hilfsstoffen bzw. den vollständigen Verzicht auf nicht notwendige Hilfsstoffe ein optimales Wirkstoff-Hilfsstoff-Mengenverhältnis bzw. Wirkstoff-Antioxidans-Verhältnis auf, sodass der Wirkstoff mit bezogen auf das Gesamtgewicht der Zusammensetzung hohem Anteil, ausreichend stabilisiert vorliegt und gleichzeitig eine pharmazeutisch akzeptable Auflösungsgeschwindigkeit aufweist.

Durch die Beigabe einer von der ersten antioxidativ wirksamen Substanz (A1) verschiedenen zweiten antioxidativ wirksamen Substanz (A2) kann die Zerfallsneigung der wenigestens einen pharmazeutisch wirksamen Substanz der BCS-Klasse II weiter gesenkt werden. Der Anteil der zweiten antioxidativ wirksamen Substanz (A2) kann dabei jedoch besonders niedrig gehalten werden. Durch ganz oder teilweisen Verzicht auf redundante Maßnahmen kann so eine Reduzierung der Herstellungskosten für die erfindungsgemäße Zusammensetzung bei gegenüber bekannten Zusammensetzungen vergleichbarer Wirkstoffstabilität gewährleistet werden. Die erfindungsgemäße Zusammensetzung und eine daraus gebildete Darreichungsform zeichnet sich durch einen gegenüber den aus dem Stand der Technik bekannten Zusammensetzungen erhöhten Anteil an pharmazeutisch wirksamer Substanz aus. Durch die insgesamt verringerte Größe der Darreichungsform und die verringerte Anzahl an nötigen Medikamentengaben wird die Patienten-Compliance erhöht. Aufgrund des erhöhten Statinanteils in der Zusammensetzung kann auf einen Kombinatonswirkstoff verzichtet und die Anzahl möglicher Nebenwirkungen reduziert werden.

Aufgrund der Vermeidung unnötiger und/oder unnötig hoher Anteile pharmazeutischer Hilfsstoffe kann, neben der Reduzierung von Nebenwirkungen, auch das Gesamtgewicht der bevorzugt festen pharmazeutischen Zusammensetzung reduziert und folglich der mögliche prozentuale Anteil des Wirkstoffes an der pharmazeutischen Zusammensetzung erhöht werden. Aufgrund des verringerten Gewichtes lässt sich auch eine Verringerung der Größe bzw. des Volumens der Darreichungsform erzielen. Dies steigert wiederum die Patienten-Compliance.

Die Erfindung sieht vor, dass eine zweite, von der ersten antioxidativ wirksamen Substanz (A1) verschiedene, antioxidativ wirksame Substanz (A2) in der pharmazeutischen Zusammensetzung Verwendung findet. Die Verwendung einer zweiten antioxidativ wirksamen Substanz hat Auswirkungen auf die Zerfallsrate der pharmazeutisch wirksamen Substanz. Die Hinzunahme einer weiteren antioxidativ wirksamen Substanz (A2) verbessert die Haltbarkeit und Stabilität der pharmazeutisch wirksamen Substanz weiter, ohne die Wirkstoffauflösungsgeschwindigkeit nachteilig zu beeinflussen.

Insbesondere pharmazeutisch wirksame Substanzen der biopharmazeutischen Klasse II sind anfällig für oxidative Degradation. Erfindungsgemäß ist vorgesehen, dass die pharmazeutisch wirksame Substanz aus dem wasserunlöslichen, oxidativ degradierbaren Statin Simvastatin besteht.

Die pharmazeutische Zusammensetzung gemäß der vorliegenden Erfindung hat überraschend gezeigt, dass die oxidative Degradation des genannten Statins in der erfindungsgemäßen pharmazeutischen Zusammensetzung bereits deutlich reduziert werden kann, wenn die pharmazeutisch wirksame Substanz mit einer ersten antioxidativ wirksamen Substanz in den vorstehend beschriebenen Mengenanteilen formuliert wird, ohne dass eine zweite antioxidativ wirksamen Substanz verwendet wird. Weiter gesenkt und auf ein Minimum reduziert wird die Degradationsneigung jedoch durch die Hinzunahme einer zweiten antioxidativ wirksamen Substanz, die von der ersten verschieden ist. Durch Kombination von zwei antioxidativ wirksamen Substanzen kann deren absoluter Anteil an der Zusammensetzung gesenkt werden.

Als besonders günstig hat sich gezeigt, wenn die pharmazeutisch wirksame Substanz Simvastatin ist. Hier wird durch Verzicht auf eine redundante antioxidative Maßnahme das Gesamtgewicht der pharmazeutischen Zusammensetzung verringert und der relative Anteil an pharmazeutisch wirksamer Substanz in der Gesamtzusammensetzung erhöht. Gleichzeitig werden aufgrund des verringerten Gewichts und dem Verzicht auf zusätzliche antioxidative Maßnahmen die Kosten der pharmazeutischen Zusammensetzung entscheidend gesenkt und die Compliance erhöht. Löslichkeit und Stabilität des Simvastatins in der erfindungsgemäßen Formulierung entsprechen denen bekannter Simvastatin-Formulierungen oder übertreffen diese sogar.

Die erste antioxidativ wirksame Substanz (A1) ist erfindungsgemäss Butylhydroxyanisol (BHA).

Erfindungsgemäß ist die zweite antioxidativ wirksame Substanz (A2) in der pharmazeutischen Zusammensetzung Zitronensäure. Hieraus ergibt sich dann eine pharmazeutische Zusammensetzung, in der neben der pharmazeutisch wirksamen Substanz eine erste antioxidativ wirksame Substanz, der Kettenterminator Butylhydroxyanisol und eine zweite antioxidativ wirksame Substanz, Zitronensäure Verwendung findet. Die Kombination aus erster und zweiter antioxidativ wirksamer Substanz mit dem Wirkstoff wie oben beschrieben, bewirkt überraschend eine weitere Absenkung der Degradationsneigung bzw. eine Verringerung des Zerfalls der pharmazeutisch wirksamen Substanz(en) in der Zusammensetzung und erlaubt relativ zu einem höheren Wirkstoffanteil insbesondere auch die Senkung des Anteils an Butylhydroxyanisol in der Zusammensetzung.

Die Erfindung sieht vor, dass die pharmazeutische Zusammensetzung als Tablette gepresst vorliegt. Um die Einnahme der Tablette weiter zu erleichtern, weist diese eine Oberflächenbeschichtung (Film-beschichtete Tablette) auf.

Eine vorteilhafte Ausführungsform der pharmazeutischen Zusammensetzung sieht vor, dass die Zusammensetzung als Filmtablette vorliegt.

Die Erfindung umfasst ebenfalls die pharmazeutische Zusammensetzung wie vorstehend beschrieben zur Verwendung in der Medizin. Insbesondere geeignet ist die erfindungsgemäße pharmazeutische Zusammensetzung als HMG-CoA-Reduktasehemmer, bevorzugt ist eine Verwendung zur Behandlung von Fett-Stoffwechsel-Störungen.

### Beispiel 1: Herstellung der pharmazeutischen Zusammensetzung zur oralen Darreichung gemäß der vorliegenden Erfindung

Es wird eine Feuchtgranulierung von pharmazeutisch wirksamer Substanz, mikrokristalliner Cellulose, Stärke und Lactose-Monohydrat durchgeführt. Als erste antioxidativ wirksame Substanz wird Butylhydroxyanisol verwendet, das in Ethanol gelöst wird. Die verwendete antioxidativ wirksame Säure wird in Wasser gelöst. Eine Standard-Wirbelschicht-Trocknungsvorrichtung wird verwendet, um einen Trocknungsverlust von 2 bis 2,5 % der getrockneten Granulate zu erzielen. Die getrockneten Granulate werden mit Magnesiumstearat gemischt und die endgültige Mischung in einem Fassmischer (bin blender) durchgeführt. Die Tablettierung erfolgt in einer Standardrundläuferpresse mit herkömmlichen bikonvexen Pressstempeln (Durchmesser 7 bis 11 mm, abhängig vom Tablettengewicht) mit einfacher Bruchkerbe mit flachen Radien. Die gebildeten Tabletten sind für eine Filmbeschichtung geeignet. Eine HPMC (Hydroxypropylmethylcellulose) basierende Beschichtungssuspension wird hergestellt. Die Menge an Lacktrockensubstanz, die notwendig ist, um den Tablettenkern zu bedecken, wird empirisch ermittelt.

Die im vorgenannten Verfahren hergestellten Tabletten weisen die in der nachfolgenden Tabelle 1 (Tab. 1) angegebene Zusammensetzung mit den gezeigten Gewichtsanteilen auf. Es werden Tabletten mit einem Tablettengewicht der beschichteten Tablette von 200 mg hergestellt. Der Anteil an pharmazeutisch wirksamer Substanz liegt bei den Tabletten bei 40 mg. Die erfindungsgemäße Zusammensetzung und die daraus gebildeten Darreichungsformen sind somit, bezogen auf das Gesamtgewicht, bei erhöhtem Wirkstoffanteil kleiner und können somit einfacher geschluckt werden als die aus dem Stand der Technik bekannten Darreichungsformen mit gleicher Wirkstoffmenge. Als pharmazeutisch wirksame Substanz wird Simvastatin verwendet.

**Table 1**

| Komponenten der Tablette | Gewicht-% der beschichteten Tablette |
|---|---|
| Simvastatin | 20.0 |
| Stärke | 20.0 |
| Lactose Monohydrat | 30.7 |
| Mikrokristalline Cellulose | 25.0 |
| Magnesiumstearate | 0.9 |
| Butylhydroxyanisol | 0.04 |
| Zitronensäure | 1.4 |
| Film-Beschichtung | 2.0 |
| Gesamtgewicht der beschichteten Tablette | 200 mg |

### Beispiel 2: Analytische Verfahren/Stabilitätstestung

Zerfallstests wurden unter Verwendung von Na-Phosphat/SDS-Puffer pH 7.0 durchgeführt und mittels HPLC unter Verwendung von UV-Detektion analysiert. Verunreinigungen wurden mittels HPLC unter Verwendung von Gradientenelution und Verwendung eines NaH2PO4-Puffers pH 4.5/ Acetonitril analysiert. Die Tabletten wurden bei 25°C/60% rF, 45°C/75% rF und zusätzlich bei 60°C/75% rF für 4 Wochen gelagert.

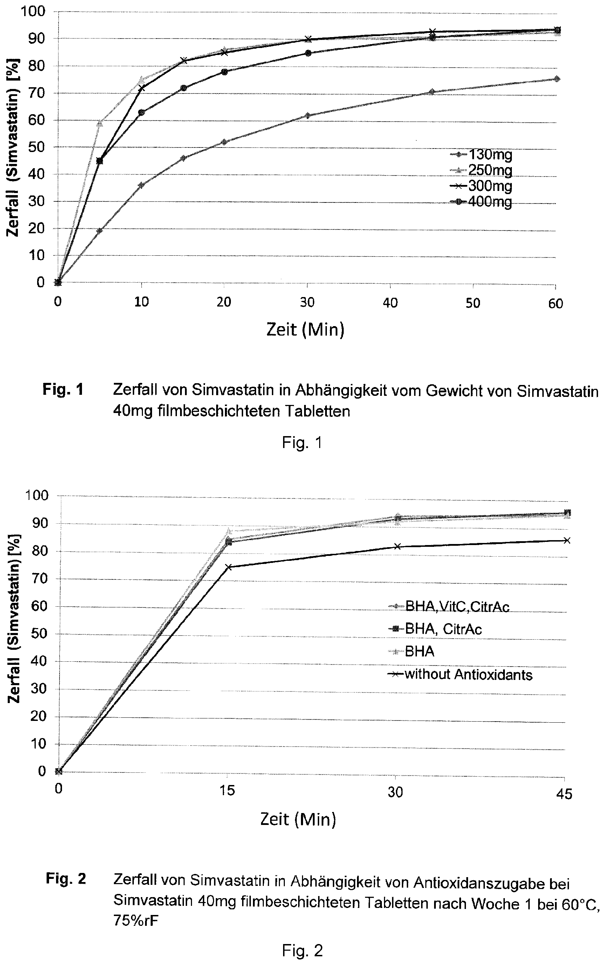

Fig. 1 zeigt das Verhältnis zwischen Tablettengewicht und Zerfall der pharmazeutisch wirksamen Substanz.
Fig. 2 zeigt die Ergebnisse nach 1 Woche Lagerung bei einer Temperatur von 60°C und 75% relativer Luftfeuchtigkeit (rF).

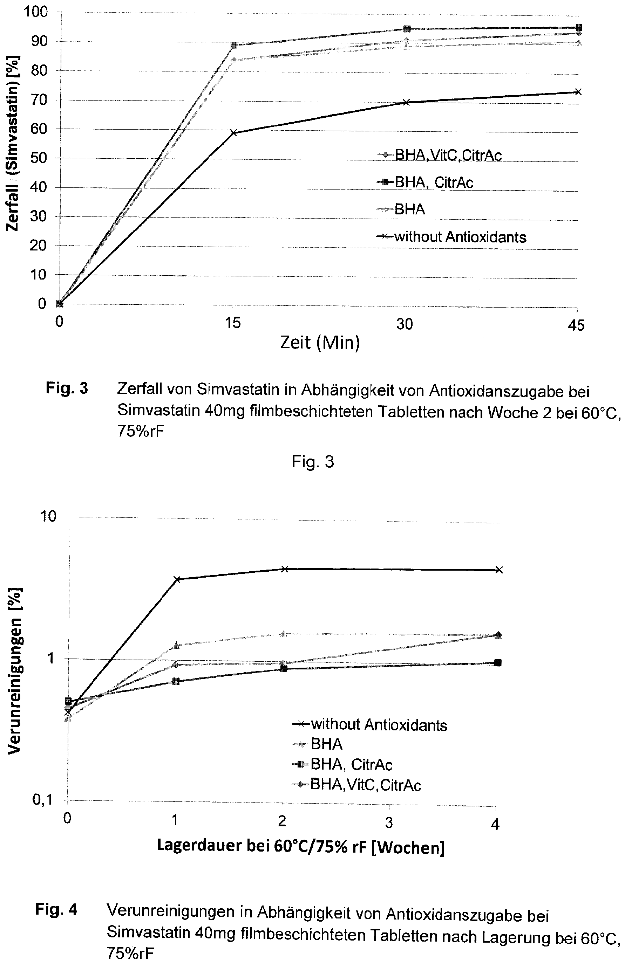

Fig. 3 zeigt, dass die Kurven ab der 2. Woche Lagerung klarer unterscheidbar werden.
Fig. 4 zeigt eine Analyse der Verunreinigungen. Geringe Unterschiede in den Zerfallsdaten können durch diese Verunreinigungsanalyse genauer untersucht werden.

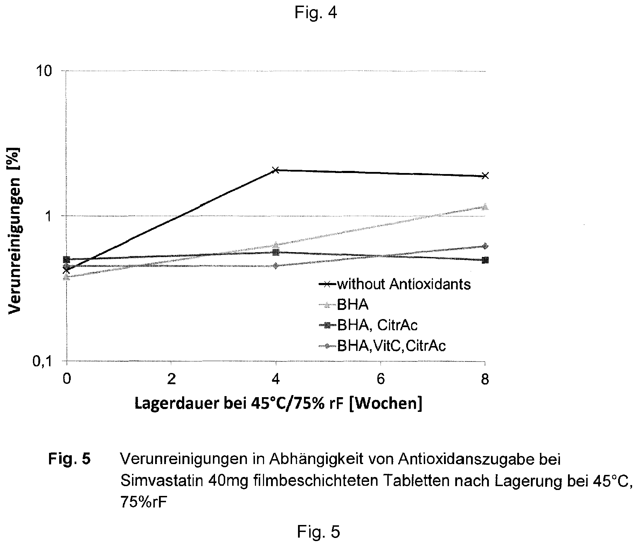

Fig. 5 stellt die Analyse der Verunreinigungen dar und bestätigt die Werte der zuvor gezeigten Fig. 4. Die Werte wurden in einer standardisierten beschleunigten Stabilitätstestung (ASII, 45°C/75% rF) ermittelt.

### Legende zu den Figuren:

BHA: Butylhydroxyanisol
VitC: Vitamin C (Ascorbinsäure)
CitrAc: Zitronensäure
without antioxidants: ohne Antioxidanzien

Aus den Figuren ist ersichtlich, dass die erfindungsgemäßen pharmazeutischen Zusammensetzungen, wie vorstehend definiert, verglichen mit herkömmlichen Zusammensetzungen vergleichbare oder verbesserte Stabilitätswerte aufweisen. Überraschend wurde festgestellt, dass die unterste Grenze für das Gewicht von Tabletten mit 40 mg Simvastatin als Inhaltsstoff, bevorzugt zwischen 250 mg und 130 mg Tablettengrundgewicht liegt. Der Wirkstoff zeigt hierbei sowohl die höchste Stabilität als auch eine hinreichende Auflösungsgeschwindigkeit.

## Patentansprüche

1. Pharmazeutische Zusammensetzung zur oralen Darreichung bestehend aus:
(i) 20 Gewichts-% Simvastatin,
(ii) 0,04 Gewichts-% Butylhydroxyanisol,
(iii) 1,4 Gewichts-% Zitronensäure,
(iv) 20 Gewichts-% Stärke
(v) 30.7 Gewichts-% Lactose Monohydrat,
(vi) 25.0 Gewichts-% Mikrokristalline Cellulose,
(vii) 0.9 Gewichts-% Magnesiumstearat,
(viii) 2 Gewichts-% Filmbeschichtung;
wobei die pharmazeutische Zusammensetzung als Tablette gepresst vorliegt und die Tablette ein Gesamtgewicht von 200 mg aufweist.

2. Pharmazeutische Zusammensetzung nach Anspruch 1 zur Verwendung in der Medizin, insbesondere als HMG-CoA-Reduktasehemmer, bevorzugt zur Behandlung von Fettstoffwechselstörungen.

## Claims

1. Pharmaceutical compound for oral administration consisting of:
(i) 20% by weight of simvastatin,
(ii) 0.04% by weight of butyl hydroxyanisole,
(iii) 1,4 % by weight of citric acid,
(iv) 20 % by weight of starch
(v) 30.7 % by weight of lactose monohydrate,
(vi) 25.0 % by weight of microcrystalline cellulose,
(vii) 0.9% by weight of magnesium stearate,
(viii) 2% by weight of a film coating;
wherein the pharmaceutical composition is in the form of a pressed tablet and the tablet has a total weight of 200 mg.

2. A pharmaceutical compound according to claim 1 for use in medicine, in particular as an HMG-CoA reductase inhibitor, preferably for the treatment of lipid metabolic disorders.

## Revendications

1. Composition pharmaceutique pour l'administration orale constitué de :
i) 20 % en poids de simvastatine,
(ii) 0,04 % en poids de butylhydroxyanisole,
iii) 1,4 % en poids d'acide citrique,
iv) 20 % en poids d'amidon ou de fécule
v) 30,7 % en poids de lactose monohydraté,
vi) 25,0 % en poids de cellulose microcristalline,
vii) 0,9 % en poids de stéarate de magnésium,
viii) 2 % en poids d'une pellicule de revêtement ;
dans laquelle la composition pharmaceutique se présente sous la forme d'un comprimé pressé et le comprimé a un poids total de 200 mg.

2. Composition pharmaceutique selon la revendication 1 pour utilisation en médecine, en particulier comme inhibiteur de l'HMG-CoA réductase, de préférence pour le traitement des troubles métaboliques lipidiques.
